Europäisches Patentamt

European Patent Office

Office européen des brevets

(11) Publication number: **0 178 220 B1**

(12) **EUROPEAN PATENT SPECIFICATION**

(45) Date of publication of patent specification: **02.01.92**  (51) Int. Cl.⁵: **C12N 15/86**, C12N 7/00, C12N 5/00

(21) Application number: **85401914.8**

(22) Date of filing: **01.10.85**

(54) Retroviral vector.

(30) Priority: **01.10.84 GB 8424757**

(43) Date of publication of application:
**16.04.86 Bulletin 86/16**

(45) Publication of the grant of the patent:
**02.01.92 Bulletin 92/01**

(84) Designated Contracting States:
**AT BE CH DE FR GB IT LI LU NL SE**

(56) References cited:
**WO-A-86/00922**

**MOLECULAR AND CELLULAR BIOLOGY, vol. 3, no. 12, December 1983, pages 2180-2180, American Society for Microbiology; A.L. JOYNER et al.: "Retrovirus transduction: generation of infectious retroviruses expressing dominant and selectable genes is associated with in vivo recombination and deletion events"**

**PROCEEDINGS OF THE NATIONAL ACADEMY OF SCIENCES OF THE USA, vol. 81, November 1984, pages 7137-7140; J.L.R. RUBENSTEIN et al.: "Construction of a retrovirus**

**capable of transducing and expressing genes in multipotential embryonic cells"**

(73) Proprietor: **INSTITUT PASTEUR**
**25-28, rue du Docteur Roux**
**F-75724 Paris Cédex 15(FR)**

Proprietor: **CENTRE NATIONAL DE LA RE-CHERCHE SCIENTIFIOUE (CNRS)**
**15, Ouai Anatole France**
**F-75007 Paris(FR)**

(72) Inventor: **Jacob, François**
**15, rue de Condé**
**F-75006 Paris(FR)**
Inventor: **Nicolas, Jean-François**
**39, rue de la Source**
**F-78570 Noisy Le Roi(FR)**
Inventor: **Rubenstein, John**
**35, rue Lecourbe**
**F-75015 Paris(FR)**

(74) Representative: **Gutmann, Ernest et al**
**S.C. Ernest Gutmann - Yves Plasseraud 67, boulevard Haussmann**
**F-75008 Paris(FR)**

Rank Xerox (UK) Business Services

## Description

The invention relates to a retroviral vector capable or transducing and expressing genes in embryonic cells, to a process for constructing such retroviral vector and to a method of transformation of said embryonic cells to confer on them the capability of expressing a determined gene or to the contrary of inhibiting the expression of an endogenous gene. The invention is applicable with particular advantage to embryonic cells which either lack a corresponding endogenous gene or which contain such an endogenous gene, whose expression is however totally or partly inhibited. The invention is also applicable to the cells themselves which contain a retroviral sequence including a determined gene, said retroviral sequence being integrated in the genomes of said cells.

It will of course be understood that the word "gene" as used hereafter, particularly when reference is made to a gene contained in the vector, must be understood as relating not only to a "natural" gene sequence cleaved from a natural DNA, but also to any nucleotidic sequence coding for a determined polypeptide, no matter how the sequence was obtained. The latter may for instance have been obtained by chemical synthesis or consist of a cDNA. Thus the word "gene" is also intended to encompass any sequence which can be transcribed into a RNA and preferably too translated into a polypeptide.

Reference will also be made hereafter by bracketted numbers to bibliographical references. The latter are recited at the end of the present specification and are incorporated herein by reference.

The following abbreviations will also be used throughout the whole specification : LTR, long terminal repeat ; EC, embryonal carcinoma ; neo, neomycin ; M-MuLV, Moloney Murine Leukemia Virus ; gRNA, genomic RNA ; env, envelope ; pol, polymerase ; gag : gene coding for the viral-structure proteins ; SVtk, SV40 early-herpes simplex I thymidine kinase promoter ; APH(3')II, aminoglycoside 3'-phosphotransferase ; β-gal, β-galactosidase.

Microinjection is the only method which has up to now been used successfully to introduce DNA into cells of the embryo and recently proper developmental regulation of microinjected genes has been obtained (1). An alternative to this technique would be to use viral vectors to transduce genes into embryonic cells. Retroviruses are an attractive choice for several reasons. The only structures required to produce recombinant genomic RNA (gRNA) from proviral DNA are grouped at the 5' and 3' termini of the provirus. These structures include : the long terminal repeats (LTR), which are required for integration (2), transcription of the gRNA and polyadenylation (3) ; the first intron which contains the packaging signal (4, 5). Furthermore, a trans-complementing cell line has been constructed which efficiently produces virions containing only the recombinant gRNA (5). Finally, the integrated recombinant virus can be reisolated by infecting the cell with a wild-type retrovirus (6).

However, although retroviruses can infect embryonic cells and integrate into the chromosome (7), there is a block in their gene expression in both embryos (8) and embryonal carcinoma (EC) cells (9). This block may be at the level of transcription (10) and/or RNA maturation.

The object of the invention is to overcome these difficulties, more particularly to provide a retroviral vector which can lead to the production of high titers of a virus which is itself capable of transmitting and expressing a determined gene into embryonic cells, particularly a gene foreign to (or heterologous with respect to) the retroviral DNA, such virus being furthermore capable of causing said determined gene to be stably expressed in said embryonic cells.

Another object of the invention is also to provide a retroviral vector capable of causing an heterologous DNA sequence to be expressed in differentiated cells (or cells in culture infectable by the corresponding virus.

Still another object of the invention is to provide a retroviral vector for the transformation of cells of high eucaryotes particularly mamalian cells, no matter whether they are in the embryonic or differentiated stage, to make them capable of producing large amounts of a corresponding retrovirus, particularly of a retrovirus containing a determined gene inserted in its genome. Thus the invention also concerns the so transformed cells.

The vector of the invention is a DNA recombinant containing a retroviral sequence including the long terminal repeats (LTR) of the 5' and 3' termini of a provirus corresponding itself to a retrovirus selected from those which are capable of infecting at least one cell species of higher eucaryotes and at least that part of the first intron which contains the packaging signal of said retrovirus (i.e. the part of the viral genome presumed to interact with a virion protein to direct specifically the packaging of the retrovirus RNA) characterized in that it further contains, downstream of said intron part in the direction of transcription, a heterologous (with respect to the virus genome) promoter region, including at least one promoter and enhancer sequence associated therewith, said promoter being selected from among those which are recognized by the endogenous polymerases of said cell species.

2

The vector constructed provides for a particularly efficient transformation of cells of higher eucaryotes and, when it further contains a determined gene downstream from the abovesaid promoter region but within an area of said recombinant sensitive to the action of said enhancer sequence, for the effective transmission and stable expression of said gene into said cell species.

When said promoter and said enhancer are respectively powerful enough, the constructed vector of this invention is capable of causing said determined gene to be stably expressed even in embryonic cells transformed by said DNA recombinant, particularly when the vector consists of a retrovirus comprising the essential elements of the above defined retroviral sequence.

The efficiency of said promoter region can be further enhanced if a second eucaryotic promoter is inserted in said DNA recombinant downstream of said first mentioned promoter, and within an area which can be activated by the enhancer sequence associated with the first promoter. It goes without saying that the second promoter must also be selected from among those which are recognized by the cell species sought to be transformed by said DNA recombinant.

A particularly preferred DNA recombinant retroviral vector contains the promoter region including the enhancer and promoter associated with the viral early region of the SV40 herpes simplex-I virus. This preferred DNA recombinant is characterized :

- in that it contains retroviral sequences derived from a retrovirus selected from those which are capable of infecting at least one species of said determined cells, and comprising the long terminal repeats (LTR) of the 5' and 3' termini of the provirus and at least that part of the first intron which contains the packaging signal ;
- in that said SV40 enhancer is located downstream of said part of the first intron in the direction of transcription and
- in that said retroviral vector contains a second eucaryotic promoter downstream of said SV40 enhancer and in an area that can be activated by said SV40 enhancer, said second promoter being selected from among those which are recognized by the endogenous polymerases of said one species of determined cells and which are sensitive to the activating action of the SV40 enhancer.

For a definition of the "SV40 enhancer" reference can be made to the article of J. BANERJI et al titled "Expression of a β-Globin Gene is Enhanced by Remote SV40 DNA Sequences" published in Cell (1981), Vol. 27, p. 299.

It is recalled that the SV40 enhancer sequence comprises some peculiar structural features, i.e. a 17 bp segment containing only the bases adenine and thymine, two repeated-sequence motifs of 21 bp and two repeated sequences of a 72 bp repeat. Promoter information for the transcription of the viral early region and probably also the late region is present within a 300 bp segment extending over position 0 from about position 5,200 to about position 300 (numbering of nucleotide positions is according to TOOZE. J, (1980), "DNA tumor viruses" (Cold Spring Harbor, New York, Cold Spring Harbor Laboratory)). Reference is more particularly made to the DNA sequences around 200 bp upstream from the initiation site transcription of the early genes in a region of two directly repeated 72 bp sequence motifs.

It is of course understood that the expression "SV40 enhancer" extends also to any sequence derived from the preceeding one by mutations or deletions, to the extent where the latter do not remove the enhancement capacity of the sequence so modified. Particularly it flows from J. BANERJI et al that the ability of SV40 deletion mutants to act as long-distance "enhancers" of a gene transcription is directly related to the presence of at least one intact 72 bp element. It may for instance consist of a mutant in which one of the 72 bp repeated motifs had been previously eliminated by deletion of the small SphI-SphI restriction fragment (positions 131 to 202).

Other promoter regions, particularly that containing the first above mentioned promoter and enhancer associated therewith, can be substituted for the SV40 region if it is as efficient as or even more efficient than the latter and it if meets the other conditions of compatibility with the contemplated cell species. For instance a promoter region which can be substituted for the SV40 promoter region is the promoter region of rat-β-actine disclosed by NUDEL et al, 1983, of the metallomethionine, of the MMTV virus, etc..

Any adequate second eucaryotic promoter may be inserted downstream from the SV40 enhancer, provided it is sensitive to the enhancing action of the SV40 enhancer and recognized by the polymerases of the host in which the DNA recombinant of this invention is to be introduced. Thymidine kinase promoters are preferred inasmuch as they are recognized by polymerase of mammalian cells of different species (mouse, chimpanzee, humans). Thymidine kinase promoters are well documented. Reference may be made more particularly to F. COLBERE-GARAPIN et al (1981), J. Mol. Biol., 150, 1-14 or F. COLBERE-GARAPIN et al (1983), The Embo J., 21-25.

Of course the use of other "second eucaryotic promoters" can be contemplated. There may be mentioned for instance the rat-β-actine- or of metallomethionine-promoter (which is inducible by heavy metals, whereby its operation can be monitored) the promoter normally associated with the HBs antigen in the Hepatitis B virus, promoters of the polyome-associated viruses, of the adeno-viruses of the vaccine-virus, etc..

The second eucaryotic promoter is not necessarily adjacent to the promoter region including said first promoter, particularly to the SV40 enhancer. Intermediate DNA sequences may be present to the extent where they do not interfere to a substantial extent with the enhancing action say of the SV40 enhancer on said second promoter and on the gene placed under the control of the latter. Needless to say however that the closer the second promoter to the SV40 enhancer, the better its enhancing action on the gene to be transcribed and expressed.

Therefore the second eucaryotic promoter is preferably adjacent to the first promoter region, particularly to said SV40 enhancer. A preferred combination of promoters is thus formed of the SV40 early-herpes simplex I-thymidine kinase promoter (SVtk) (11).

An advantageous use of the recombinant vector is for the stable transformation of cell cultures of high eucaryotes, particularly of the mammalian cells, as the combination into a same recombinant vector of the two LTRs of a retrovirus and of a promoter region as defined above positionned in between provides for a replication-, integration-, and expression-system which is particularly efficient in the corresponding cells.

The recombinant vector may be either in the form of an infectious recombinant or of a plasmid including most of the retroviral sequence defined hereafter in relation to the retrovirus, said retroviral sequence being itself recombined with a plasmid sequence including the replicon thereof for replication and amplification thereof in the suitable host cell, for instance a bacterium, such as E. coli.

More particularly the recombinant vector of the invention is a virus vector when its DNA is limited to the retroviral DNA structure essentially bonded by the LTRs, the promoter region as defined above, including the intron part or whole intron containing the packaging signal, the enhancer sequence and one or preferably two tandemly-linked promoters (identical to or different from each other) and at least a gene position downstream from the heterologous promoter region and upstream from the 3'LTR. Said gene portion may consist of the natural ones, i.e. gag, pol and env, or part thereof, more particularly the final part of the env gene in the direction of transcription, or preferably another gene substituted at least in part for the nucleotidic sequence comprising up to the gag, pol and first part of the env gene in the direction of transcription, said another gene being a foreign or heterologous gene the expressioon of which is sought in a high eucaryotic cell, particularly at the embryonic stage thereof.

As a matter of fact preferred recombinant DNAs of the invention, particularly in the form of the infectious retroviruses are capable not only of transmitting into embryonic cells (or embryos), a gene (initially positionned in the retroviral DNA recombinant under the control of said promoter region, particularly of said second promoter and within the area of enhancing action of the enhancer associated with the first promoter, such as the SV40 enhancer, but also of providing for the steady and stable expression of said gene throughout cell generations and subsequent to the differentiation thereof.

Thus a preferred use of the retroviral DNA of the invention is the transformation or infection of embryonic cells at the earliest stages of their development, say between the second up to the eighth division.

The invention further relates to a process for producing a recombinant vector according to the invention, starting from a plasmid or analogous vector containing a retroviral sequence including the LTRs and all endogenous sequences of the retrovirus that are necessary for the replication and encapsidation thereof in a cell host infectable by the corresponding retrovirus, which process comprises cleaving said retroviral sequence or deleting a part of said viral sequence downstream from the intron sequence containing the packaging signal and upstream of the terminal part of the env gene (and the 3'LT$^R$) and inserting therein or substituting therefor a promoter region as above defined and, whenever appropriate, the gene or genes the exporession of which will later be sought. The different heterologous parts to be inserted in the retrovirus (or substituted for the endogenous parts thereof which are to be deleted) can of course be achieved in one or several steps, as will appear most convenient depending on the restriction sites available in the area concerned of the retrovirus.

The invention further comprises a process for making an infectious virus vector, comprising transforming with the preceding DNA recombinant a competent cell, i.e. a cell capable of producing a retrovirion corresponding to the retroviral sequence of said DNA recombinant and containing at least a defective retroviral DNA integrated in its genome capable of trans-complementing the retroviral sequence of the DNA recombinant.

Preferred cells used for this process are retrovirus trans-complementing cell lines, i.e. cells which contain a defective retroviral DNA which does not contain the intron sequence containing the packaging signal, which however contains that part of the retroviral DNA sequence which is no longer present in the retroviral DNA recombinant introduced in the cell, particularly the sequence containing the gag, pol and env genes. When the DNA recombinant of the invention contains a retroviral sequence derived of M-MuLV, preferred competent cells, for the production of an infectious retrovirus vector, are for instance NIH 3T3 cells which had been transformed with a defective M-MuLV DNA, having a Ball-PstI deletion (351 bp) between the 5'LTR and the start codon for Pr65$^{gag}$ , more particularly 6 bp upstream from the presumed donor site for the env mRNA splice, and approximately 50 bp from the start codon for Pr65$^{gag}$ : see particularly reference (5).

Cell infected with a non-defective retrovirus, particularly a wild retrovirus can be used too. However upon transformation of these cells with the DNA recombinant plasmid disclosed hereafter, the so transformed cells will produce both wild virions and infectious recombinant viruses according to the invention. The presence of the wild virus may be without later consequence, if not harmful to the cells to be infected by the recombinant virus of the invention and modified by the gene sought to be stably expressed by the infected cells.

The capability of the recombinant virus vectors obtained of infecting the corresponding recipient cells and of causing integration and expression of a gene more particularly in embryonic cells can be detected upon using any dominant marker, such as the neo gene which has been used in the examples hereafter, downstream and under the control of the above defined promoter region. The use of the dominant marker also provides the test to be used in assaying the effectiveness of any enhancer-promoter region or any combination of enhancer and sets or promoters inserted in the retroviral sequence of a retrovirus vector liable of being used to cause the transmission and stable expression of the marker - or any other gene - in embryonic cells.

Additional features of the invention will appear as a more detailed disclosure of DNA recombinants and properties thereof proceeds, more particularly in connection with the drawings in which :

Fig. 1 is a diagram of a recombinant in accordance with the invention and Fig. 2 a diagram of another recombinant constructed for comparative purposes.

Fig. 3 is an autoradiograph of an in situ assay of APH(3')II (the neo gene-encoded phosphotransferase) after electrophoretic separation from the endogenous phosphotransferase activity as obtained after transformation of different cell lines with plasmids or direct infection of said cell lines with infection recombinant viruses containing the essential elements defined above in accordance with the invention.

The different elements which are brought into play in the recombinants which will be disclosed hereafter were selected inasmuch as they consist of models which, as is well known to the specialists, are recognized as being susceptible of extrapolation to other systems.

Particularly the observations that can be made on embryonal carcinoma cells (EC) when the latter are subjected to determined treatments, can be extrapolated with a great degree of certainty to other embryonic cells, particularly to mammalian cells (of animals or humans). This susceptibility of extrapolation is highly documented.

The same applies to the Moloney Murine Leukemia Virus. The results that are obtained upon transforming or modifying the structure of this virus and the results achieved thereby can be held as highly representative of what could be observed under similar conditions with other retroviruses, transformed or modified accordingly and then used under conditions substantially similar to those which will be disclosed hereafter in connection with the M-MuLV retrovirus.

Finally the gene coding for APH(3')II provides a good model for the determination of the behavior of genes after introduction into a cell by means of a retroviral recombinant as described hereafter, owing to its capability of being used as a dominant hybrid selective marker for higher eucaryotic cells.

Construction of the retroviral vectors :

1) plasmid pMMuLV-SVtk-neo.

The starting DNAs used were plasmid pB6 (12) and vector pSVtk-neo$\beta$ (11).

Plasmid pB6 was itself derived from plasmid pMOV3 (13) which contained a proviral copy of M-MuLV. A SalI site had been constructed by inserting the M13mp8 polylinker (14) into the PstI site at position 563 of the M-MuLV/gRNa (15) contained in pMOV3. The BamHI site, which is at position 6,537 of the M-MuLV/gRNA, is in the 5' end of the env gene.

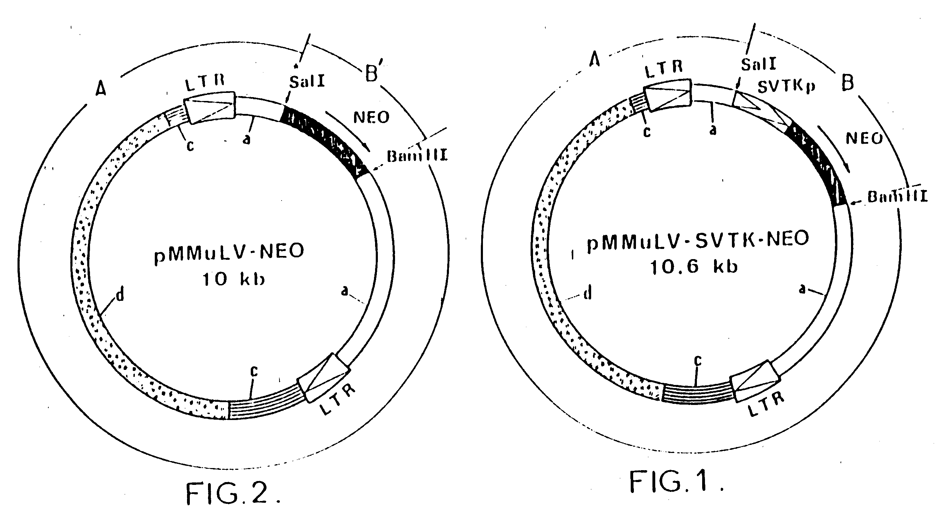

Plasmid pB6 was digested with the restriction SalI and BamHI and the fragment including the two LTR (identified by arc A in Fig. 1) was ligated to fragment identified in Fig. 1 by arc B (fragment B).

Fragment B had itself been obtained as follows. pSVtk-neo$\beta$ was digested with BamHI and HindIII. The BamHI-HindIII fragment which contained the SVtk region was then ligated to a fragment containing a SalI site and obtained from plasmid pCH110 (20) by digestion thereof with BamHI (complete digestion) and HindIII (partial digestion) to provide the pCH110-SVtk-neo-recombinant. The latter plasmid was then cleaved by SalI and BamHI to provide the abovesaid fragment B (SalI-SVtk-neo-BamHI).

The recombinant obtained pM-MuLV-SVtk-neo had a size of 10.6 kb.

Another plasmid pM-MuLV-neo, having a size of 10 kb was also constructed for comparative purposes. It was obtained by ligating a fragment A' including the 5'LTR and 3'LTR and obtained from pB6 with a B'fragment obtained from pSVtk-neoβ.

More particularly fragment A' had been obtained by linearizing pB6 with SalI, repairing the staggered extremities with polymerase I to provide blunt extremities, cleaving further with BamHI and recovering as fragment A' the fragment including the two LTRs. Fragment B' was obtained by cleaving pSVtk-neoβ with BglII, repairing the staggered ends by polymerase I cleaving further by BamHI and recovering the fragment containing the neo gene and freed from the Svtk region.

Procedures used in the construction of these vectors are referenced in RUBENSTEIN and CHAPPELL (16). The enzymes used in the plasmid construction were obtained from BOEHRINGER MANNHEIM.

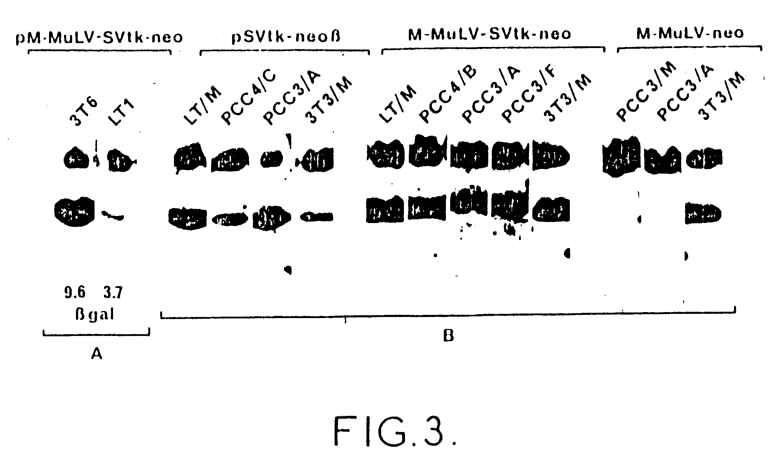

The plasmids obtained include parts of different origins diagrammatized in different manners in Fig. 2 and 3, particularly (when not directly identified in the drawings) :

(a) M-MuLV sequences ;

(b) neo gene (the arrow corresponds to the orientation of the coding strand of the gene) ;

(c) mouse chromosomal DNA ;

(d) pBR322 sequences : * : the SalI site in pM-MuLV-neo was destroyed during the construction of this molecule.

There are a number of features of the structure of these retroviral vectors molecules which warrant further description. (i) There are two 492 base pair LTRs which are essential for transcription of the genomic RNA and integration of the provirus (2, 3). (ii) 3' to the 5' LTR are 418 base pairs (up to position 563 of the gRNA, see ref. 15) which contain M-MuLV's first intron (15) and the putative packaging signal of the genomic RNA (5). (iii) The following SalI recognition site is derived from the M13mp8 bacterial phage polylinker (14). (iv) Between this SalI site and the BamHI site (position 6,537 in the M-MuLV gRNA) is the 1,100 base pair neo gene. The neo gene can confer G418 resistance to mammalian cells (11, 24). One of the plasmids (pM-MuLV-SVtk-neo has the SV40 early and herpes simplex I thymidine kinase promoters (600 base pairs (11)) 5' to the neo gene. (v) Following the neo gene are 1,237 base pairs corresponding to the 3' end of the M-MuLV env gene, followed by the 3' LTR which contains the polyadenylation signal (15). (vi) Flanking both LTRs are sequences derived from the mouse chromosome (200 bp 5' and 800 bp 3') (12). (vii) The remaining 4.4 kilobases of the plasmid are sequences corresponding to a rearranged copy of pBR322, which expresses the ampicillin resistance gene. Therefore, the neo gene in pM-MuLV-neo (Fig. 2) is expected to be under the transcriptional control of only the LTR, whereas in pM-MuLV-SVtk-neo the neo - (Fig. 1) gene is expected to be under the transcriptional control of the LTR, the SV40 early promoter, and the thymidine kinase promoter.

Cell culture, virus infection and G418 selection :

Cell lines :

All cells were grown in Dulbecco's modified Eagle's medium (DMEM) containing 10 % fetal calf serum, penicillin and streptomycin. The mouse EC cell lines utilized were LT-1, PCC4Aza$^{R1}$ and PCC3 HPRT$^-$Ouab$^R$ (17). The mouse differentiated cell lines were φ2 (5), NIH 3T3 (18), and NIH 3T6.

Virus production :

φ2 cells containing recombinant retroviral DNA were placed in 6 cm diameter culture dishes containing 4 ml of medium without G418. The cell density was approximately at 50 % confluency. After incubating the cells at 37°C for 24 hours, the medium was removed and then it was centrifuged at 3000xg at 4°C for 15 minutes.

Virus infection :

One milliliter of virus stock, containing 5 μg/ml of polybrene (Sigma) was added to 5 x 10$^5$ cells in 6 cm plates. The samples were incubated at 37°C for 2 hours and then the medium was replaced with fresh medium. One day after the viral infection (or DNA transformation, see next section), various numbers of

cells (between $5 \times 10^5$ to $2.5 \times 10^4$) were transferred to 10 cm plates. One day after this transfer, the medium was replaced with medium containing 500 μg/ml of G418 (Geneticin from Gibco) and was changed every 2 days until only G418 resistant cells were present, at which point the medium was changed every 3 days. The G418 resistant colonies were counted at 10 to 14 days after the beginning of the G418 selection. Individual clones were obtained by sucking them up into a 2 ml pipette and transferring them into 1 cm culture wells.

DNA transformation :

Plasmid DNA was transfected into cells using the technique of Graham and Van der Eb (19). The calcium-phosphate precipitate was formed using 20 μg of DNA in a volume of 500 μl. In acute transfections, and most of the stable transfections, 6.6 μg of the plasmid pCH110 was added to the other DNA as an internal control for the efficiency of transfection. this plasmid expresses β-galactosidase (20). The DNA precipitates were added to a 6 cm plate containing $5 \times 10^5$ cells. Then, after incubating the cells for 30 minutes at 22°C, 5 ml of medium was added. One day later, the medium was changed, and then, after an additional 24 hours, protein extracts were prepared from the cells. For stable transformation experiments, the cells were dilute, into 10 cm plates containing fresh medium after the initial 24 hours (see the previous section).

Assay of protein extracts for APH(3')II and β-galactosidase :

Cells were washed twice using TS (138 mM NaCl, 5 mM NaCl, 5 mM KCl, 0.7 mM $Na_2HPO_4$, 25 mM Tris Base, 0.7 mM $CaCl_2$ and 1.0 mM $MgCl_2$, pH 7.4). Then, after adding 200 μl of lysis buffer (85 % TS, 15 % glycerol, 10 mM dithiothreitol) the cells were scraped off the plates and sonicated (Ultrasonics Inc. W-375) for 2 minutes at 4°C, using 100 % power output and 50 % duty cycle settings. Next, 1/10 volume of 0.5 % deoxycholate and 1.0 % NP40 was added. The cellular debris was pelleted by centrifugation for 10 minutes in a Beckman microfuge, and the supernatant was saved. The protein concentration of the extract was assayed using the Bradford procedure (21). The activity of β-galactosidase and of APH(3')II were assayed according to references (22) and (23) respectively.

The retroviral vector containing the internal SVtk promoter expresses the neo gene in EC cells :

Transient expression :

To test whether unintegrated copies of a retroviral vector can express the neo gene, we transfected pM-MuLV-SVtk-neo DNA into fibroblast cells (NIH 3T6) and EC cells. (LT-1). Fig. 3 reveals that the recombinant retroviral DNA leads to the production of detectable quantities of APH(3')II in both cell lines, although pM-MuLV-SVtk-neo is expressed five-fold more efficiently in the NIH 3T6 cells than in the LT-1 cells.

Stable transformants :

Next we tested whether the recombinant retroviral vectors could stably express the neo gene in EC cells (presumably after chromosomal integration). We transfected LT-1 cells with pM-MuLV-neo or pM-MuLV-SVtk-neo DNA. Table I which reports the efficiency of producing G418 resistant clones, shows that only the retroviral vector containing the SVtk promoter has the ability to produce G418 resistant LT-1 cells, although both plasmids are equally efficient at transforming differentiated cells (see next section). Next, to learn whether adjacent retroviral sequences have an effect on the expression of the SVtk-neo gene in EC cells, we compared the efficiency of transformation of pM-MuLV-SVtk-neo to that of pSVtk-neoβ, a non-retroviral plasmid which also uses the SVtk promoter to express the neo gene. As shown in Table I, these two plasmids are essentially equally efficient at confering G418 resistance to LT-1 cells. therefore, it is unlikely that a mutation is required for pM-MuLV-SVtk-neo to be transcriptionally active in EC cells.

Production of viruses from a recombinant retroviral plasmid containing an internal SVtk-neo transcription unit :

To produce virions carrying gRNA encoded by pM-MuLV-neo or pM-MuLV-SVtk-neo, we transfected these plasmids into ψ2 cells, a retrovirus trans-complementing cell line, which does not produce M-MuLV, but can package recombinant retroviruses (5). Following the plasmid transfection, cells expressin the neo gene were selected by adding G418 to their culture medium. The efficiency of obtaining G418 resistant clones was approximately $10^{-3}$ for both plasmids (Table I).

The transformed clones were mixed, and their supernatant was collected. We tested whether this supernatant contained retroviruses carrying the neo gene, by exposing NIH 3T3 cells to the supernatant and then selecting for cells capable of growing in G418. The supernatants from the $\psi2$ cells carrying the pM-MuLV-neo or the pM-MuLV-SVtk-neo plasmids had a tier of approximately $8 \times 10^5$ G418 resistant colony-forming units per milliliter (G418$^R$ cfu/ml) (Table I). Therefore, $\psi2$ cells containing these retroviral vectors are efficient producers of virions which can transfer neo gene into differentiated cells. Furthermore, the internal SVtk promoter does not substantially change the efficiency of producing these recombinant retroviruses (Table I). Finally, both M-MuLV-neo expressed the same level of APH(3')II in infected NIH 3T3 cells (Fig. 1 and 2).

Viral transduction of the neo gene into EC cells :

We next tested whether the M-MuLV-neo and the M-MuLV-SVtk-neo viruses could confer G418 resistance to EC cells. Three EC cell lines were studied, each having different developmental properties. LT-1 is a nullipotent cell line, whereas PCC3 and PCC4 are multipotent (17). These EC cells were grown in the virus-containing medium, and the cells were then transferred into medium containing G418. In contrast to the result obtained with differentiated cells (NIH 3T3), only the M-MuLV-SVtk-neo virus was capable of confering stable G418 resistance to all three types of EC cells. The M-MuLV-neo virus was either completely unable to transduce an active neo gene (when infecting LT-1 cells), or very inefficient relative to the pM-MuLV-SVtk-neo virus (when infecting PCC4 or PCC3 cells) as shown in Table I.

Table I. Efficiency of producing G418 resistant cells using the recombinant retroviruses

| | (A) Virus (G418$^R$ Cfu/ml) | | (B) DNA | | |
|---|---|---|---|---|---|
| | M-MuLV-neo | M-MuLV-SVtk-neo | pM-MuLV-neo | pM-MuLV-SVtk-neo | pSVtk-neoβ |
| ψ2 | — | — | $11 \times 10^{-4}$ | $8 \times 10^{-4}$ | — |
| NIH 3T3 | $9 \times 10^{5}$ | $6.5 \times 10^{5}$ | — | — | — |
| LT-1 | 0 | $2 \times 10^{2}$ | 0 | $3 \times 10^{-5}$ | $6 \times 10^{-5}$ |
| PCC4 | 10 | $2.8 \times 10^{2}$ | — | — | — |
| PCC3 | $4 \times 10^{2}$ | $2 \times 10^{3}$ | — | — | — |

The table reports the number of G418 resistant colonies obtained from one milliliter of ψ2 supernagtant (G418$^R$ Cfu/ml).

The efficiency of producing G418$^R$ colonies is reported as the ratio of the number of G418$^R$ colonies divided by the number of cells initially transfected.

We isolated individual G418 resistant EC clones, all of which maintained their typical EC morphology. To prove that the neo gene is actually expressed in these cells, we assayed cellular extracts for APH(3')II.

Fig. 2 is an autoradiograph of the in situ assay of APH(3')II (the neo gene-encoded phosphotransferase) after electrophoretic separatioon fromthe endogenous phosphotransferase activity. The arrow points to the position of the APH(3')II activity.

(A) Extracts from NIH 3T5 and LT-1 cells were assayed fro APH(3')II 48 hours after co-transfection with pM-MuLV-SVtk-neo and pCH110 DNA. We used the level of $\beta$-galactosidase ($\beta$-gal) expressed from the pCH110 as an internal control for the efficiency of transfection. The numbers 9.6 and 3.7 are the number of $\beta$-gal units in the NIH 3T6 and LT-1 extracts, respectively. the definition of $\beta$-gal units can be found in reference 32.

(B) APH(3')II assay of extracts from G418[R] LT-1, PCC3, PCC4 and NIH 3T3 cells, which had received the neo gene by DNA transformation (pSVtk-neo$\beta$) or viral infection (M-MuLV-SVtk-neo and M-MuLV-neo). Individual clones (A, B, C and F) or mixtures of many clones (M) were assayed.

Fig. 3 shows the results of this assay, which demonstrates that the neo gene product is expressed in all of the G418 resistant cells infected with the M-MuLV-SVtk-neo virus. On the other hand, the G418 resistant PCC3 cells derived from infection with the M-MuLV-neo virus contained at least 40 fold less APH(3')II than EC cells infected with the M-MuLV-SVtk-neo virus, or differentiated cells infected with either the M-MuLV-neo or M-MuLV-SVtk-neo virus, or differentiated cells infected with either the M-MuLV-neo or M-MuLV-SVtk-neo virus.

Therefore, the addition of the internal SVtk promoter 5' to the neo gene confers up on retroviruses the ability to transduce and stably express the neo gene in nullipotent and multipotent EC cells. Furthermore, the few G418 resistant EC cells transduced by M-MuLV-neo produce 40 fold less APH(3')II than M-MuLV-neo transduced differentiated cells.

The results obtained thus show that the retrovirus (M-MuLV-SVtk-neo) was capable of introducing and expressing the neo gene in all EC cell lines tested. Each of these EC cell lines has characteristics of different stages of embryonic development (1), suggesting that this retroviral vector may be suitable for introducing and expressing genes in embryos.

On the other hand, the retrovirus without the SVtkpromoter (M-MuLV-neo) is unable, or extremely inefficient at expressing the neo gene in EC cells. For instance, using either DNA transformation or viral infection (table 1), pM-MuLV-neo was unable to confer stable neo gene expression to LT-1 cells. In contrast to LT-1 cells, PCC4 and PCC3 cells were transformed to G418 resistance by the M-MuLV-neo retrovirus, although at an extremely low efficiency (10 and 400 G418[R] cfu/ml respectively, table 1). These G418 resistant cells maintain EC morphology and multipotency. Although they are resistant to G418, their level of APH(3')II expression is at least 40 fold lower than in the EC cells which had been infected with M-MuLV-SVtk-neo (Fig. 1). Therefore, the retroviral promoter and splicing are probably functioning in these cells, but at a reduced efficiency. One of the characteristics of PCC3 cells is their ability to differentiate in culture. For this reason, it is likely that whithin the PCC3 cell population, cells at different states of differenciation coexist (28). We hypothesize that a sub-population of PCC3 cells have the ability to express, although inefficiently, genes under the transcriptional control of the retroviral LTR.

The fact that the internal SVtk promoter did not significantly change the amount of virus produced by the $\psi$2 cells containing pM-MuLV-SVtk-neo (Table I) shows that a second promoter, which is in tandem with the LTR, is not necessarily deleterious to the propagation of a retrovirus.

It will of course be understood that any other gene or nucleotidic sequence can be substituted for the neo gene, particularly when the expression of said other gene or nucleotidic sequence is sought.

The vector of the invention can also be used to transduce an antiparallel copy of a gene into a cell. We (32) and another group (33) have presented evidence that antiparallel mRNA can inhibit the expression of the complementary mRNA in eukaryotic cells. The inhibition of a developmentally important gene, by expressing an antiparallel copy of that gene, has the potential of being a powerful technique to study development.

In the preceding example the gene transmitted into and expressed by the embryonic cells was the neo gene. It goes without saying that said gene can be substituted by any other gene, particularly the gene the expression of which is desired in the embryonic cells. For instance the pM-MuLV-SVtk-neo can be digested by SalI and BgIII, and the retroviral fragment obtained containing the LTRs and the promoter region yet freed of the neo gene, be ligated to the extremities of the gene concerned, if necessary after appropriate modification of extremities of said retroviral fragment and of said gene by any of the classical methods useful for that purpose (for instance by forming blunt ends or using of synthetic intermediate linkers provided with the adequate restriction sites, etc.). This is of course but an example of the different possible manners of providing for the introduction of a determined gene under the control of the SVtk promoter region of pM-MuLV-SVtk-neo.

The preceding vector is moreover particularly adapted for the transformation of mouse cells. It goes without saying that the basic principle of the invention applies also particularly to other types of cells. It is also worthwhile mentionning that the host specificity of a given strain may be modified, for instance by transforming the cells of a different host, say human cells, however first caused to contain at least part of the genome of a xenotypic-type retrovirus, particularly part of the env gene of said xenotypic-type virus, the latter being selected among those whose env genes are capable of transcomplementing the env gene of the mouse-adapted retrovirus.

The invention further concerns the high eucaryotic cells, particularly mamalian cells and even more particularly embryonic cells which contain, integrated in their respective genomes a retroviral sequence as above defined, said retroviral sequence comprising the gene whose expression is sought in said cells. For instance the cells of the invention contain the β-globin gene or cloned human HPRT genes, for use in embryonic cells otherwise defective as regards these particular genes. The invention concerns particularly congelated modified cells of that type or cells of that type maintained in any other form of preservation.

The invention is also for use in the veterinary field. Particular it authorizes for the modification of animal embryonic cells, in order to insert in their genomes additional genes capable of expressing determined proteins (or of supplementing the expression of natural endogenous proteins). These inserted genes may also have functions of regulation of endogenous genes, to increase - or to the contrary to repress - their expression. Such regulating functions can be useful for instance in causing an increase of the meat production in cattle or poultry or of milk production in cows. Thus the invention relates to embryonic cells modified according to the invention, in a suitable state of preservation, for instance in the congelated form, for implantation in the uterus of a female animal using any suitable technique.

Repression of a gene may for instance be achieved upon introducing an antiparallel copy of said gene by means of a recombinant vector according to the invention in the cells concerned. it has been shown that an anti-parallel mRNA can inhibit theexpression of the complementary mRNA in eucaryotic cells (RUBENSTEIN et al (1984) C.R. Hebd. Séances Acad. Sci. Ser III 299, 271-274, and SOUTHERN, P.J. et al (1982), J. Mol. Appl. Genet. I. 327-342).

The use of the invention is of course not limited to the modification of embryonic cells. It can also be contemplated for the modification of differenciated cells in animals or even in humans. For instance the DNA recombinant vector of this invention can be used for the modification of hematopoietic cells (normally present in the bone-marrow) first taken up from a mammal with a view of being re-injected in said mammal, for instance when the latter has, in the meantime, undergone a treatment, i.e. radioactive irradiation. The use of the invention is also contemplated to overcome genetic deficiencies in mammals, for instance in HGPRT-defective mammals (Lesh Nyan disease in man). In a same manner as above described, competent cells from such mammals can be taken up from said mammals, be modified by a recombinant DNA vector according to the invention modified by a HGPRT + gene and re-injected into said mammals.

M-MuLV-SVtk-neo virus has been deposited at the "Collection Nationale des Cultures de Micro-organismes" (Pasteur Institute of Paris, France) under Number I-341 on September 25, 1984.

1. Brinster, R.L., Ritche, K.A., Hammer, R.E., O'Brien, R.L., Arp, B. & Storb, U. (1983) Nature 306, 332-336.
2. Panganiban, A.T. & Temin, H.M. (1983) Nature 306, 155-160.
3. Weiss, R.A., Teich, N., Varmus, H.E., Coffin, J.M., Eds. (1982) The Molecular Biology of Tumor Viruses. Part III. RNA tumor viruses. (Cold Spring Harbor Laboratory, Cold Spring Harbor, New York).
4. Watanabe, S. & Temin, H.M. (1982) Proc.Natl. Acad.Sci.USA 79, 5986-5990.
5. Mann, R., Mulligan, R.C. & Baltimore, D. (1983) Cell 33, 153-159.
6. Perkins, A.S., Kirschmeier, P.T., Gattoni-Celli, S. & Weinstein, I.B. (1983) Mol.Cell.Biol. 3, 1123-1132.
7. Jaenisch, R., Fan, H. & Croker, B. (1975) Proc.Nat. Acad.Sci.USA 72, 4008-4012.
8. Jaenisch, R., Jähner, D., Nobis, P., Simm, I., Löhler, J., Harbers, K. & Grotkopp, D. (1981) Cell 24, 519-529.
9. Periés, J., Alves-Cardoso, E., Canivet, M., Debons Guillemin, M.C. & Lasneret, J. (1977) J.Natl.Cancer Inst. 59, 463-465.
10. Linney, E., Davis, B., Overhauser, J., Chao, E. & Fan, H. (1984) Nature 308, 470-472.
11. Nicolas, J.F. & Berg, P. (1983) In "Cold Spring Harbor Conferences on Cell Proliferation. Vol. 10:Teratocarcinoma Stem Cells (Cold Spring Harbor Laboratory, Cold Spring Harbor, New York) pp.469-485.
12. Yermanian, P. (1984) Thèse de Doctorat en Médecine, Université Paris XII.
13. Harbers, K., Schnieke, A., Stuhlmann, H., Jähner, D. & Jaenisch, R. (1981) Proc.Nat. Acad.Sci.USA 78, 7609-7613.
14. Messing, J. & Vieira, J. (1982) Gene 19, 269-276.
15. Shinnick, T.M., Lerner, R.A. & Sutcliffe, J.G. (1981) Nature 293, 543-548.
16. Rubenstein, J.L.R. & Chappell, T.G. (1983) J.Cell Biol., 96, 1464-1469.
17. Nicolas, J,F., Jakob, H. & Jacob, F. (1981) In "Functionally differentiated cell lines". G. Sato ed. Alan R. Liss Publ., New York, 185-210.

18. Fan, H. & Paskind, M. (1974) J.Virol. 14, 421-429.

19. Graham, F.L. & van der Eb, A.J. (1973) Virology 52, 456-467.

20. Hall, C.V., Jacob, P.E., Ringold, G.M. & Lee, F. (1983) J.Mol.Appl.Genet. 2, 101-109.

21. Bradford, M. (1976) Anal.Biochem. 72, 248-254.

22. Miller, J.H. (1972) Experiments in Molecular Genetics. Cold Spring Harbor Laboratory, Cold Spring Harbor, New York.

23. Reiss, B., Sprengel, R., Will, H. & Schäller, M. (1984) Gene (in press).

24. Scuthern, P.J. & Berg, P. (1982) J.Mol.Appl. Genet. 1, 327-342.

25. Stewart, C.L., Stuhlman, H., Jähner, D. & Jaenisch, R. (1982) Proc.Nat.Acad.Sci.USA 79, 4098-4102.

26. Gautsch, J.W. & Wilson, M.C. (1983) Nature 301, 32-36.

27. Niwa, O., Yokota, Y., Ishida, H. & Sugahara, T. (1983) Cell 32, 1105-1113.

28. Nicolas, J.F., Dubois, P., Jakob, H., Gaillard, J. & Jacob, F. (1975) Ann.Microbiol. 126A, 3-22.

29. Shimotohno, K. & Temin, H. (1981) Cell 26, 67-77.

30. Tabin, C.J.,Hoffmann, J.W., Goff, S.P. & Weinberg, R.A. (1982) Mol.Cell.Biol. 2, 426-436.

31. Joyner, A.L. & Bernstein, A. (1983) Mol.Cell. Biol. 3, 2180-2190.

## Claims

**1.** A DNA recombinant vector which can be used for transducing an embryonic eucaryotic cell with a foreign gene encoding a RNA, so that said embryonic cell will stably transcribe said gene into said RNA; said vector being characterized by:

a) long terminal repeats of the 5' and 3' termini of a provirus corresponding to a retrovirus capable of infecting said embryonic cell;

b) a packaging signal of a first intron of said provirus; said packaging signal being downstream of said 5' long terminal repeat in a direction of transcription of said vector; and

c) a first promoter and an enhancer which are heterologous to said retrovirus and are downstream of said packaging signal; said first promoter being recognizable by an endogenous polymerase of said cell;

d) said first promoter, said enhancer and said 5' long terminal repeat being so located that said foreign gene, which is heterologous to said retrovirus, can be located in said vector: i) downstream of said first promoter and said enhancer and ii) under the transcriptional control of said 5' long terminal repeat and said first promoter.

**2.** The vector of claim 1 which also comprises a second promoter recognizable by an endogenous polymerase of said cell; said second promoter being: i) heterologous to said retrovirus, ii) downstream of said first promoter and said enhancer and iii) within an area of said vector sensitive to the action of said enhancer, so that said foreign gene can be located downstream of, and under the transcriptional control of, said second promoter.

**3.** The vector of claim 1 or 2 wherein said first promoter and said enhancer are both from an SV40 gene, from a B-actine gene, from a metallomethionine gene or from an MMTV gene.

**4.** The vector of claim 2 or 3 wherein said second promoter is a thymidine kinase promoter, a B-actine promoter, a metallomethionine promoter, a hepatitis B virus surface antigen promoter, a polyoma virus promoter or an adenovirus promoter.

**5.** The vector of anyone of claims 1-4 wherein said retrovirus is an M-MuLV retrovirus.

**6.** The vector of anyone of claims 1-5 wherein said enhancer is an SV40 enhancer.

**7.** The vector of anyone of claims 2-6 wherein said first promoter and said enhancer are from an SV40 early region and said second promoter is a thymidine kinase promoter.

**8.** The vector of anyone of claims 1-7 which also comprises a replicon for replication and amplification in E. coli.

**9.** The vector of anyone of claims 1-8 which further comprises said foreign gene.

10. The vector of anyone of claims 1-9 which is a recombinant retrovirus, with which said embryonic cell can be infected.

11. An embryonic eucaryotic cell transformed with the vector of anyone of claims 1-10.

12. A process for making the recombinant retrovirus of claim 10 which comprises transforming, with the vector of anyone of claims 1-9, a cell that is capable of producing a retrovirion corresponding to the recombinant retrovirus and that contains a defective retroviral DNA integrated in its genome capable of trans-complementing the vector.

13. The process of claim 12 wherein said cell is a retrovirus trans-complementing cell which does not contain an intron sequence containing a packaging signal.

14. A process for transducing and expressing, in an embryonic eucaryotic cell, a foreign gene characterized by the use of the vector of anyone of claims 1-10.

15. The process of claim 14 wherein said embryonic cell is transformed by infection with the retrovirus of claim 10.

16. The process of claim 15 wherein said embryonic cell is transformed with a recombinant retrovirus made by the process of claim 12 or 13.

**Revendications**

1. Vecteur recombinant d'ADN qui peut être utilisé pour la transduction d'une cellule eucaryote embryonnaire avec un gène étranger codant pour un ARN, de sorte que ladite cellule embryonnaire transcrira de façon stable ledit gène dans ledit ARN ; ledit vecteur étant caractérisé par :
   a) de longues répétitions terminales des extrémités 5' et 3' d'un provirus correspondant à un rétrovirus capable d'infecter ladite cellule embryonnaire ;
   b) un signal d'empaquetage d'un premier intron dudit provirus ; ledit signal d'empaquetage étant en aval de ladite longue répétition terminale en 5' dans une direction de transcription dudit vecteur ; et
   c) un premier promoteur et un activateur qui sont hétérologues audit rétrovirus et sont en aval dudit signal d'empaquetage ; ledit premier promoteur étant reconnaissable par une polymérase endogène de ladite cellule ;
   d) ledit premier promoteur, ledit activateur et ladite longue répétition terminale en 5' étant situés de telle sorte que ledit gène étranger, qui est hétérologue audit rétrovirus, peut être situé dans ledit vecteur : i) en aval dudit premier promoteur et dudit activateur, et ii) sous le contrôle de transcription de ladite longue répétition terminale en 5' et dudit premier promoteur.

2. Vecteur selon la revendication 1, qui comprend également un deuxième promoteur reconnaissable par une polymérase endogène de ladite cellule ; ledit deuxième promoteur étant : i) hétérologue audit rétrovirus, ii) en aval dudit premier promoteur et dudit activateur, et iii) à l'intérieur d'une zone dudit vecteur sensible à l'action dudit activateur, de telle sorte que ledit gène étranger peut être situé en aval, et sous le contrôle de transcription, dudit deuxième promoteur.

3. Vecteur selon la revendication 1 ou 2, dans lequel ledit premier promoteur et ledit activateur sont tous deux issus d'un gène du SV40, d'un gène de la $\beta$-actine, d'un gène de la métallométhionine ou d'un gène du MMTV.

4. Vecteur selon la revendication 2 ou 3, dans lequel ledit deuxième promoteur est un promoteur de la thymidine kinase, un promoteur de la $\beta$-actine, un promoteur de la métallométhionine, un promoteur de l'antigène de surface du virus de l'hépatite B, un promoteur du virus du polyome ou un promoteur d'un adénovirus.

5. Vecteur selon l'une quelconque des revendications 1 à 4, dans lequel ledit rétrovirus est un rétrovirus M-MuLV.

6. Vecteur selon l'une quelconque des revendications 1 à 5, dans lequel ledit activateur est un activateur du SV40.

7. Vecteur selon l'une quelconque des revendications 2 à 6, dans lequel ledit premier promoteur et ledit activateur sont issus d'une région précoce du SV40 et ledit deuxième promoteur est un promoteur de la thymidine kinase.

8. Vecteur selon l'une quelconque des revendications 1 à 7, qui comprend également un réplicon pour la réplication et l'amplification dans E. coli.

9. Vecteur selon l'une quelconque des revendications 1 à 8, qui comprend en outre ledit gène étranger.

10. Vecteur selon l'une quelconque des revendications 1 à 9, qui est un rétrovirus recombinant, avec lequel ladite cellule embryonnaire peut être infectée.

11. Cellule eucaryote embryonnaire transformée par le vecteur tel que défini à l'une quelconque des revendications 1 à 10.

12. Procédé de préparation du rétrovirus recombinant tel que défini à la revendication 10, qui comprend la transformation, par le vecteur tel que défini à l'une quelconque des revendications 1 à 9, d'une cellule qui est capable de produire un rétrovirion correspondant au rétrovirus recombinant et qui contient un ADN rétroviral déficient intégré dans son génome capable de transcomplémenter le vecteur.

13. Procédé selon la revendication 12, dans lequel ladite cellule est une cellule trans-complémentant un virus qui ne contient pas de séquence intron contenant un signal d'empaquetage.

14. Procédé de transduction et d'expression, dans une cellule eucaryote embryonnaire, d'un gène étranger caractérisé par l'utilisation du vecteur tel que défini à l'une quelconque des revendications 1 à 10.

15. Procédé selon la revendication 14, dans lequel ladite cellule embryonnaire est transformée par infection par le rétrovirus tel que défini à la revendication 10.

16. Procédé selon la revendication 15, dans lequel ladite cellule embryonnaire est transformée par un rétrovirus recombinant obtenu par le procédé tel que défini à la revendication 12 ou 13.

**Patentansprüche**

1. DNA-Rekombinations-Vektor, der zur Transduktion einer embryonalen eukaryotischen Zelle mit einem eine RNA-codierenden fremden Gen verwendbar ist, so daß die embryonale Zelle das Gen stabil in die RNA transkribiert; wobei der Vektor gekennzeichnet ist durch:
   a) "long terminal repeats" der 5'- und 3'-Enden eines Provirus, das einem zur Infektion der embryonalen Zellen fähigen Retrovirus entspricht;
   b) ein Verpackungssignal eines ersten Introns des Provirus; wobei das Verpackungssignal stromabwärts des 5'-"long terminal repeats" in Transkriptionsrichtung des Vektors liegt; und
   c) einen ersten Promotor und einen Enhancer, die zum Retrovirus heterolog sind und stromabwärts des Verpackungssignals liegen; wobei der erste Promotor von einer endogenen Polymerase der Zelle erkennbar ist;
   d) wobei der erste Promotor, der Enhancer und der 5'-"long terminal repeats" so liegen, daß das fremde Gen, das zum Retrovirus heterolog ist, im Vektor lokalisiert werden kann: i) stromabwärts vom ersten Promotor und Enhancer und ii) unter der Transkriptionskontrolle des 5'-"long terminal repeats" und des ersten Promotors steht.

2. Vektor nach Anspruch 1, der einen zweiten von einer endogenen Polymerase der Zelle erkennbaren Promotor enthält, wobei der zweite Promotor i) heterolog zum Retrovirus ist, ii) stromabwärts vom ersten Promotor und Enhancer und iii) in einem für die Wirkung des Enhancers sensitiven Bereich des Vektors liegt, so daß das fremde Gen stromabwärts davon lokalisiert werden kann, und unter der Transkriptionskontrolle des zweiten Promotors steht.

3. Vektor nach Anspruch 1 oder 2, wobei der erste Promotor und der Enhancer beide von einem SV40-

14

Gen, von einem B-Actin-Gen, von einem Metallomethionin-Gen oder von einem MMTV-Gen stammen.

4. Vektor nach Anspruch 2 oder 3, wobei der zweite Promotor ein Thymidin-Kinase-Promotor, ein B-Actin-Promotor, ein Metallomethionin-Promotor, ein Hepatitis B-Virus-Oberflächenantigen-Promotor, ein Polyoma-Virus-Promotor oder ein Adenovirus-Promotor ist.

5. Vektor nach einem der Ansprüche 1 bis 4, wobei das Retrovirus ein M-MuLV-Retrovirus ist.

6. Vektor nach einem der Ansprüche 1 bis 5, wobei der Enhancer einen SV40-Enhancer ist.

7. Vektor nach einem der Ansprüche 2 bis 6, wobei der erste Promotor und der Enhancer aus dem frühen Bereich von SV40 stammen und der zweite Promotor ein Thymidin-Kinase-Promotor ist.

8. Vektor nach einem der Ansprüche 1 bis 7, der ferner ein Replikon zur Replikation und Amplifikation in E. coli enthält.

9. Vektor nach einem der Ansprüche 1 bis 8, der ferner das fremde Gen enthält.

10. Vektor nach einem der Ansprüche 1 bis 9, der ein rekombinantes Retrovirus ist, mit dem die embryonale Zelle infiziert werden kann.

11. Embryonale eukaryotische Zelle, die mit dem Vektor nach einem der Ansprüche 1 bis 10 transformiert ist.

12. Verfahren zur Herstellung eines rekombinanten Retrovirus nach Anspruch 10, bei dem man eine Zelle, die dazu fähig ist, ein Retrovirion entsprechend dem rekombinanten Retrovirus zu bilden, und die eine defekte retrovirale DNA in ihrem Genom enthält, die den Vektor in trans komplementieren kann, mit dem Vektor nach einem der Ansprüche 1 bis 9 transformiert.

13. Verfahren nach Anspruch 12, wobei die Zelle eine in trans Retrovirus-komplementierende Zelle ist, die keine Intron-Sequenz enthält, die ein Verpackungssignal enthält.

14. Verfahren zur Transduktion und Expression eines fremden Gens in einer embryonalen eukaryotischen Zelle, gekennzeichnet durch die Verwendung des Vektors nach einem der Ansprüche 1 bis 10.

15. Verfahren nach Anspruch 14, wobei die embryonale Zelle durch Infektion mit dem Retrovirus nach Anspruch 10 transformiert wird.

16. Verfahren nach Anspruch 15, wobei die embryonale Zelle mit einem nach den Verfahren von Anspruch 12 oder 13 hergestellten rekombinanten Retrovirus transformiert wird.

FIG.1.

pMMuLV-SVTK-NEO
10.6 kb

FIG.2.

pMMuLV-NEO
10 kb

FIG.3.